# EUROPEAN PATENT SPECIFICATION

(11) **EP 3 397 329 B1**
(45) Date of publication and mention of the grant of the patent: **08.03.2023**
(21) Application number: 16825896.0
(22) Date of filing: 21.12.2016
(51) Int. Cl.: A61M 11/00

(54) **AIR-FLOW IN A NEBULIZER HEAD**
LUFTSTROM IN EINEM VERNEBLERKOPF
ÉCOULEMENT D'AIR DANS UNE TÊTE DE NÉBULISEUR

(30) Priority: 28.12.2015 US 201562271387 P
(43) Date of publication of application: 07.11.2018
(73) Proprietor: Koninklijke Philips N.V., 5656 AG Eindhoven (NL)
(72) Inventor: VON HOLLEN, Dirk Ernest, 5656 AE Eindhoven (NL); DUNCAN, Michael, 5656 AE Eindhoven (NL); QUADRELLI, Filippo, 5656 AE Eindhoven (NL)
(74) Representative: Philips Intellectual Property & Standards
(86) International application number: PCT/IB2016/057847
(87) International publication number: WO 2017/115223

(56) References cited:
- EP-A1- 1 882 487
- EP-A1- 2 735 328
- EP-B1- 1 278 569
- WO-A1-2009/128556
- US-A1- 2003 072 717
- US-A1- 2005 166 912
- US-A1- 2008 000 470
- US-A1- 2008 000 470
- US-A1- 2008 149 096
- US-A1- 2015 306 334
- US-B1- 6 412 481

## Description

### FIELD OF THE INVENTION

The present invention relates to air-flow in a nebulizer head, and relates in particular to a nebulizer head, and to a nebulizer system for providing a substance in an aerosolized form using a nebulizer.

### BACKGROUND OF THE INVENTION

For inhalation purposes, for example for inhalation therapy of applying a drug to a patient, a liquid may be used to generate a plurality of small droplets, which is also called atomization of the liquid, and to generate a mist or aerosol that can be inhaled by the patient. For the atomization of the liquid, compressed air, ultrasonic or mesh droplet generators are provided, as examples. In US 2008/0000470 A1, an inhalation therapy device is provided in which an air-flow is provided by air conveying means and liquid droplets and the air-flow are mixed in a nebulizing chamber to provide the mixture to be inhaled. After inhaling, i.e. during exhaling, aerosol droplets may still be present in the exhaled air stream. However, it has been shown that when discharging the exhaled air droplet mixture into the environment, at least for certain drug medication, care must be taken that the drug medication does not get on the face of the patient, for example into the eyes.

US 2008/000470 A1 describes an aerosol generating device 1 that comprises a respiratory air flow generating means and a nebulising chamber into which the generated liquid droplets and the respiratory air flow are supplied. The nebulising chamber comprises a tapering area which ends in a tubular intubation means. The intubation means is designed such that the intubation end can be positioned in such a manner that the liquid droplet/respiratory air mixture conveyed Via the intubation means is released behind those areas of the respiratory tract that filter out to a great extent the liquid droplets from the mixture.

US 2005/166912 A1 describes an ation structure including an inhalation/exhalation orifice. The inhaler system includes a medicament supply system having a medicament ejector disposed adj acent the inhalation/exhalation orifice. The medicament supply system controllably ejects medicament droplets from the medicament ejector. Further, the inhaler system includes a flow control system in fluidic communication with the medicament ejector.

EP 1 882 487 A1 describes an inhaler with which the user inhales the medicine through a suction port has a gas holding part in which exhaled air of the user or other air is to be stored, an air flow path which is connected with the gas holding part and guides the medicine to the suction port by inhalation of the user, and a medicine ejection part from which the medicine is to be ejected to the interior of the airflow path when the user inhales through the airflow path the exhaled air or other air stored in the gas holding part.

### SUMMARY OF THE INVENTION

There may thus be a need to provide a nebulizer head with an improved air-flow guidance.

The object of the present invention is solved by the subject-matter of the independent claim, wherein further embodiments are incorporated in the dependent claims.

According to the present invention, a nebulizer head is provided comprising a housing structure, a fluid reservoir and an aerosol generator. The housing structure provides an air-flow path with two opposite air-flow openings. The fluid reservoir is provided to accommodate a liquid from which small droplets are to be generated in order to form an aerosol. The aerosol generator has an aperture member that comprises an aperture surface with a plurality of apertures to provide the small droplets from the liquid. The fluid reservoir is arranged adjacent the aerosol generator such that the aperture member is in contact with the fluid. The aerosol generator is arranged within the air-flow path. At least one by-pass flow path is provided between the aerosol generator and the housing structure. The air-flow path is a bi-directional path that provides a first air-flow direction for inhaling and a second air-flow direction for exhaling. The air enters the air-flow path and leaves the air-flow path via the air-flow openings. The aperture surface is arranged transverse to the first and second air-flow direction.

Due to arranging the air-flow path having at least one by-pass flow path around the aerosol generator, which is arranged between the two openings, the air exits at the rear side, i.e. opposite to the face side, when the patient breathes out. During inhalation, an improved mixture is provided due to the arrangement of the aerosol generator in the air-flow pathway and the arrangement of the aperture surface such that the generated droplets are provided in the inhalation air-flow direction. The by-pass flow path reduces the re-deposition of drugs in form of the small liquids onto the aperture surface of the aerosol generator.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1A shows a schematic cross-section through a nebulizer head and the resulting air-flow and aerosol generation during inhalation;
Fig. 1B shows a respective schematic illustration of the air-flow during exhalation;
Fig. 2 shows a perspective illustration of the air-flow during inhalation;
Fig. 3 shows a vertical cross-section through the nebulizer head;
Fig. 4 shows a vertical cross-section transverse to the viewing direction of Fig. 3 through a nebulizer head;
Fig. 5 shows a schematic illustration of a nebulizer system; and
Fig. 6 shows a schematic illustration of basic steps of a method for providing a substance in an aerosolized form.

### DETAILED DESCRIPTION OF EMBODIMENTS

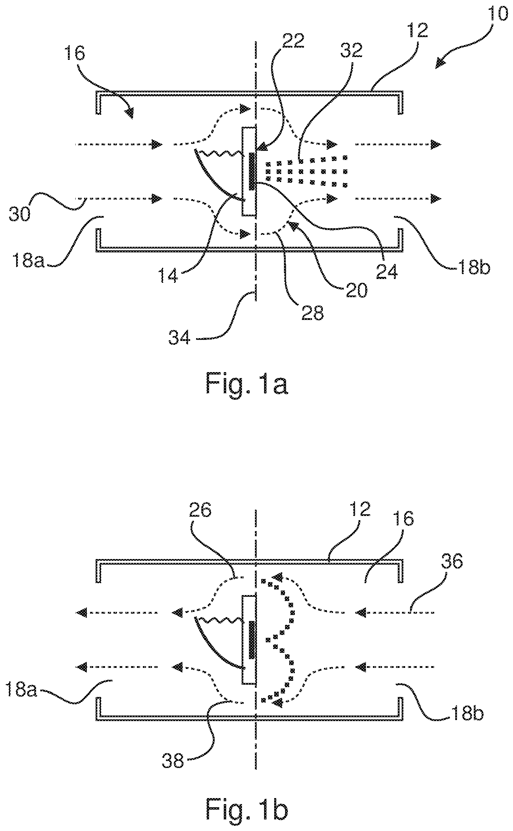

Fig. 1A shows a schematic section through a nebulizer head 10 that comprises a housing structure 12 and a fluid reservoir 14. The fluid reservoir 14 is provided to accommodate a liquid from which small droplets are to be generated in order to form an aerosol. The housing structure 12 provides an air-flow path 16 with two opposite air-flow openings 18a, 18b. Further, an aerosol generator 20 is provided that comprises an aperture member 22. The aperture member 22 is provided with an aperture surface 24 with a plurality of apertures (not further shown) to provide the small droplets from the liquid.

The fluid reservoir 14 is arranged adjacent the aerosol generator 20 such that the aperture member 22 is in contact with the fluid. The aerosol generator 20 is arranged within the air-flow path 16. The fluid reservoir 14 may be accessible from the top, for example via an opening.

At least one by-pass flow path 26 is provided between the aerosol generator 20 and the housing structure 12. The air-flow path 26 is a bi-directional path that provides a first air-flow direction 28 for inhaling. In Fig. 1A, dotted arrows 30 illustrate the resulting air-flow during the inhalation. Further dotted arrows 32 illustrate the generation of the liquid droplets. A hashed line 34 illustrates a plane, in which the aperture surface 24 may be arranged.

Fig. 1B shows the air-flow during exhalation. Dotted arrows 36 illustrate the resulting air-flow. As can be seen, the bi-directional path provides a second air-flow direction 38 for exhaling.

The air enters the air-flow path and leaves the air-flow path via the air-flow openings 18a, 18b. The aperture surface 24 is arranged transverse to the first and second air-flow direction 28, 38.

In an example, a plane, in which the aperture member is arranged, for example the mesh plane 34, is provided perpendicular to the flow direction.

The aerosol generator can also be referred to as aperture member generator.

The first air-flow direction is an inhaling or inhalation direction and the second air-flow direction is an exhaling or exhalation direction.

In an example, the air-flow is manually activatable by the user breathing in and out respectively.

An advantage of the above described arrangement is the generation of the aerosol droplets into the air-flow path having a similar direction in order to achieve a well distributed aerosol to be inhaled by the patient.

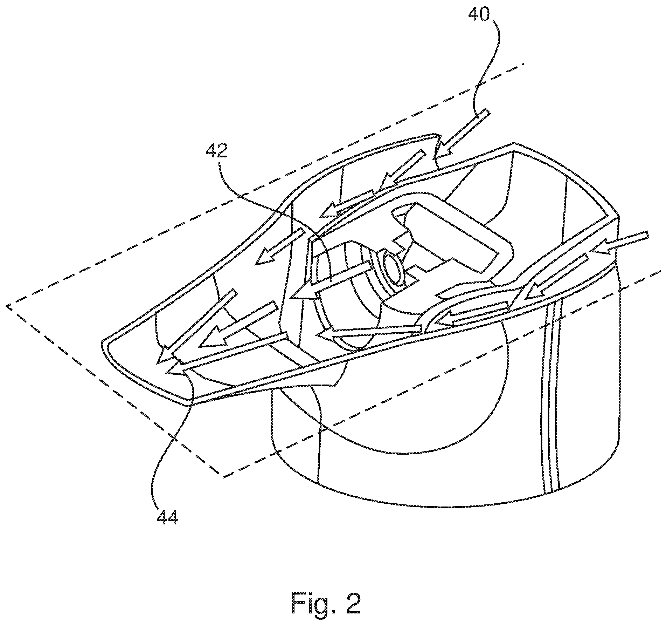

This is also indicated in Fig. 2, in which first arrows 40 indicate an air-flow during inhalation and second arrows 42 indicate the generation of the aerosol droplets to provide an aerosolized drug delivery to the patient at a mouth-piece opening 44.

During exhalation, the by-pass flow path 26 avoids the re-deposition of the aerosol droplets and rather allows a circumvention of the aerosol generators aperture surface, as indicated in Fig. 1B.

Hence, as an example, it is provided that the air-flow openings are each provided as bi-directional air inlet and air outlet openings.

As an option, it is shown to arrange the aerosol generator 20 within the air-flow path 16 such that by-pass flow paths are arranged around the aerosol generator. For example, at least a portion of the perimeter of the aerosol generator is provided with by-pass channels.

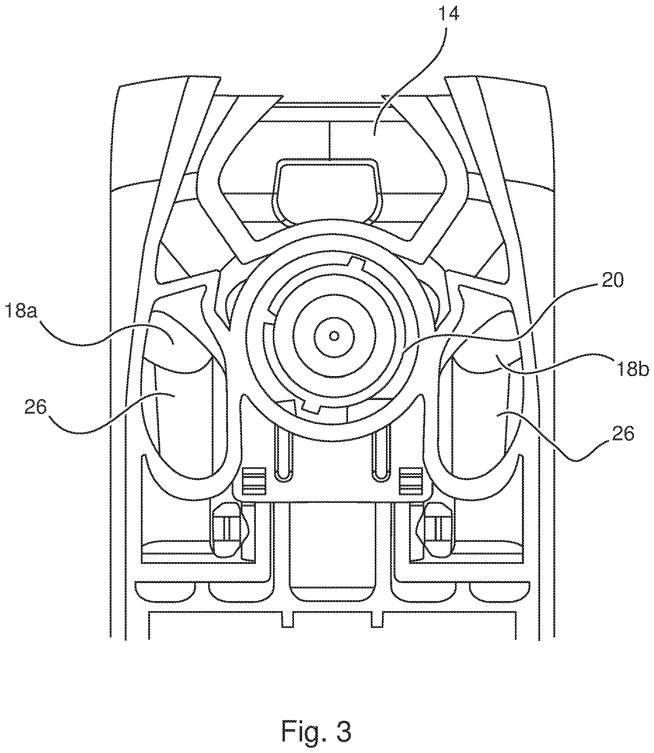

Fig. 3 indicates the aerosol generator 20 and respectively arranged by-pass flow paths 26 on either side.

In another example, not further shown, the aerosol generator 20 is provided with by-pass flow paths all around. In another example, several by-pass flow paths are provided in an approximately symmetrical manner around the aerosol generator.

In an example, the aperture member is mounted substantially vertically in the head. The term "vertically" relates to the normal use when the air-flow path is arranged horizontally.

As a further option, Fig. 3 also indicates the arrangement of the fluid reservoir 14 above the aerosol generator 20, but which fluid reservoir 14 has a downwardly reaching extension such that the liquid can be provided on the respective side of the aerosol generator.

As a further option, the by-pass flow paths 26 are arranged as upward sloping venting ducts towards the rear.

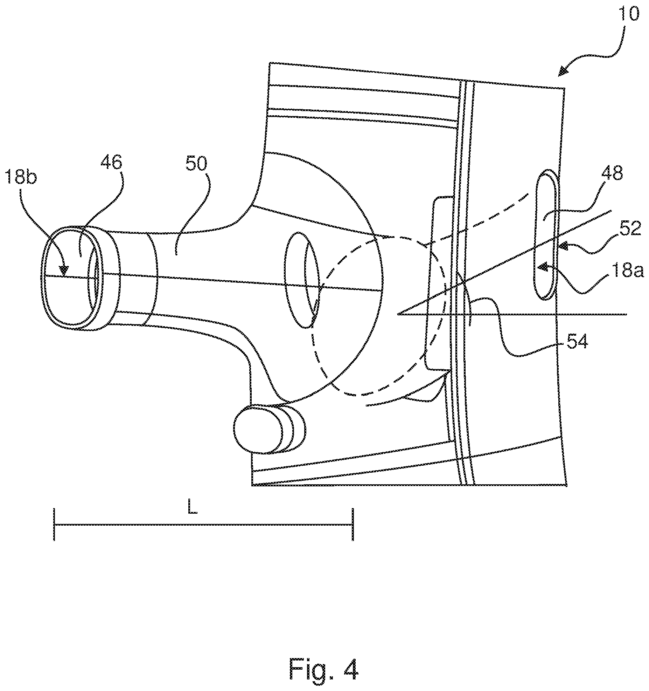

Fig. 4 shows the nebulizer head 10, and as an option the arrangement of one of the air-flow openings being provided as a mouth-piece opening 46, and the other one of the air-flow openings is provided as a backside opening 48. A flow direction from the backside opening 48 to the mouth-piece opening 46 is the first air-flow direction and the vice versa flow direction is the second air-flow direction.

As an option, as already indicated above, the aerosol generator 20 provides the small droplets in the inhaling direction, and during inhaling, a flow of air flowing around the aerosol generator 20 is provided at a rear side of the aperture surface 24.

As an option, the backside opening is arranged behind the aperture surface 24.

The backside opening is arranged downstream the aperture surface in the exhaling air-flow direction, and upstream in the inhaling air-flow direction. The backside opening is arranged at a distal end when the user is breathing through the mouthpiece.

As a further option it is indicated in Fig. 4 that a portion of the air-flow path between the aerosol generator and the mouth-piece opening is a front air channel 50 and a portion between the aerosol generator and the backside opening is a rear air channel 52. The rear air channel 52 and preferably the by-pass sections, i.e. the by-pass flow paths 26, are equal or larger in clear cross-section than the mouth-piece opening. This further improves the air-flow within the nebulizer head.

As a further option it is indicated in Fig. 4 that the rear air channel 52 is angled upward in the exhaling direction, as indicated with angle symbol 54. The air channel is thus angled upwards towards the back side opening. In a normal handling position, the mouth-piece is arranged in a horizontal manner, and the aperture member is hence in a vertical manner.

As a further option, the mouth-piece 46 is provided as a fixedly mounted mouth-piece. The mouth-piece is hence not removable.

As a further option, it is provided that the mouth-piece 46 provides the front air channel 50 having a length *L* between the mouth-piece opening and the aperture member, which length *L* is larger than 5 cm. The mouth-piece opening may be oval in shape.

The aperture member 22 may be provided as a mesh or membrane with a plurality of holes or apertures. The aperture member 22 may also be provided as a plate with a plurality of droplet outlets.

The configuration of the exit outlet or conduit in an angled manner away from the mesh surface prevents direct inline access to the mesh surface and the length is increased to ensure a finger cannot come into contact with the mesh surface. Preferably, this angle between an horizontal axis and a longitudinal axis of the mouthpiece may be greater than five degrees.

In an example, the exit conduit is angled away from the center line of the mesh and combined with the increased length, and the exit port at the mouth-piece is oval in shape. This geometry restricts the patient from accessing the mesh. The conduit may be angled greater than 5° and lengthened to a minimum of 5 cm with a minimum cord of 2 cm for the outlet oval shape.

The arrangement of the above-mentioned clear cross-section relations allows to provide a low pressure resistance to tidal breathing flow rates.

To arrange the rear airway channel angled upward also improves the situation due to dipping from condensate when the aerosol impacts the inner walls of the air channel, since the slope allows for the condensate to be captured during treatment and may then be disposed off after treatment. As an added benefit, the rear vents are positioned to minimize the action of a patient from covering the vents when holding the device.

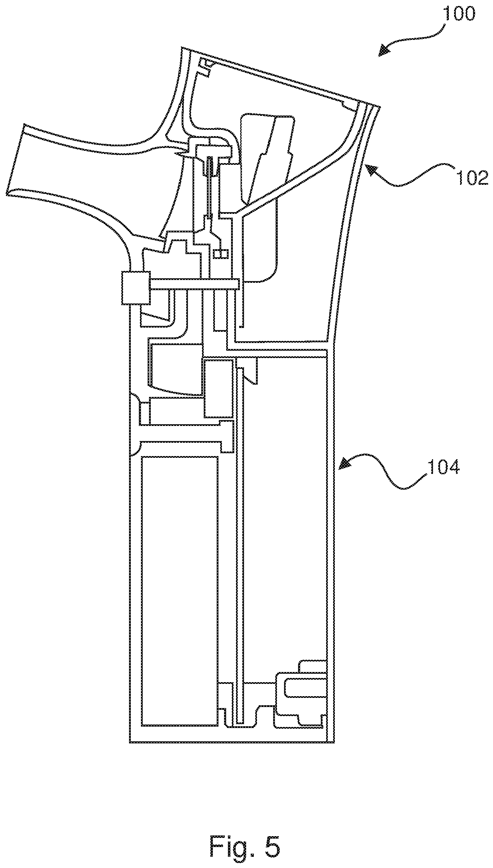

In Fig. 5, a nebulizer system 100 is illustrated in a schematic cross-section. The nebulizer system 100 comprises a nebulizer head 102 and a base structure 104. The nebulizer head 102 is provided as one of the above-mentioned examples of the nebulizer head 10. The base structure comprises a power supply and a control and operating element for operating the aerosol generator. The nebulizer head 102 is at least temporarily mounted to the base structure.

As indicated in Fig. 5, the nebulizer head is provided for a hand-held device. However, the nebulizer head may also be provided for a portable device, in which the base structure 104 would not form a hand-held grip portion, but would be a base station.

As an option, the nebulizer head may be mounted removably.

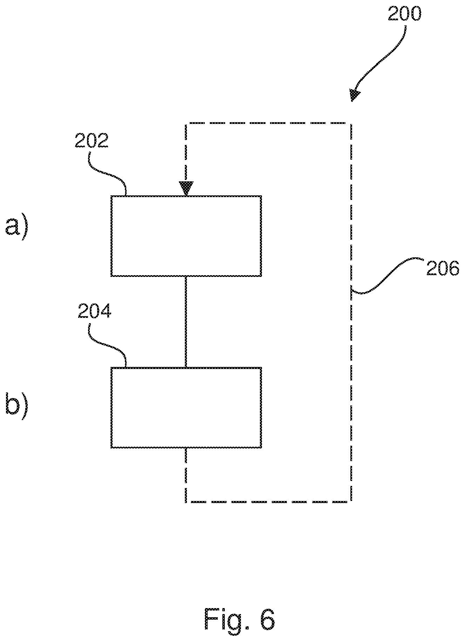

Fig. 6 shows a method 200 for providing a substance in an aerosolized form using a nebulizer. The method 200 comprises the following steps: In a first step 202, also referred to as step a), the substance is provided as aerosol droplets within an air-flow caused by inhalation of the patient. The air-flow is entering an air-flow path at a rear air-flow opening and is flowing around an aerosol generator at least partly such that the aerosol droplets mix with the air-flow to form an aerosol. The rear air-flow opening is facing away from a patient inhaling. The aerosol is exiting the air-flow path at a mouth-piece opening to enter a patient's mouth or nose for further reaching patient's air passages and respiratory tracts. In a second step 204, also referred to as step b), the exhaled air is discharged. The exhaled air is entering the air-flow path at the mouth-piece opening and is flowing around the aerosol generator at least partly such that the aerosol droplets are transported towards the rear air-flow opening. The air mixture is exiting the air-flow path at the rear air-flow opening to be discharged to the surrounding air away from the patient exhaling.

The first and second steps 202, 204 may then be repeated, as indicated with repeat loop arrow 206.

Any reference signs in the claims should not be construed as limiting the scope.

## Claims

1. A nebulizer head (10), comprising:
- a housing structure (12) providing a bidirectional air-flow path (16) between two opposite air-flow openings (18a, 18b), wherein one of the air-flow openings is provided as a mouth-piece opening and another one of the air-flow openings is provided as a backside opening, and wherein a flow direction from the backside opening to the mouth-piece opening is a first air-flow direction for inhaling and a vice versa flow direction is a second air-flow direction for exhaling;
- a fluid reservoir (14) provided to accommodate a liquid from which small droplets are to be generated in order to form an aerosol;
- an aerosol generator (20) with an aperture member (22) that comprises an aperture surface (24) with a plurality of apertures to provide the small droplets from the liquid; wherein the fluid reservoir is arranged adjacent the aerosol generator such that the aperture member is in contact with the fluid, wherein the aperture member (22) is mounted substantially vertically in the nebulizer head (10) in relation to the air-flow path (16) which is, in normal use of the nebulizer head (10), arranged horizontally;
wherein the aerosol generator is arranged within the air-flow path;
wherein at least one by-pass flow path (26) is provided between the aerosol generator and the housing structure, wherein the by-pass flow path (26) is arranged around the aerosol generator (20), and wherein a flow direction of the by-pass flow path (26) is the same as the second air-flow direction;
wherein the air enters the air-flow path and leaves the air-flow path via the air-flow openings;
wherein the aperture surface is arranged transverse to the first and second air-flow direction; and
wherein the air-flow is manually activatable by the user breathing in and out respectively; and wherein the air-flow is non-supported by ventilation means.

2. Nebulizer head according to claim 1, wherein the air-flow openings are each provided as bi-directional air inlet and air outlet openings.

3. Nebulizer head according to claim 1 or 2, wherein the aerosol generator is arranged within the air-flow path such that by-pass flow paths are arranged around the aerosol generator.

4. Nebulizer head according to one of the preceding claims, wherein one of the air-flow openings is provided as a mouth-piece opening (46) and the other one of the air-flow openings is provided as a backside opening (48); and a flow direction from the backside opening to the mouth-piece opening is the first air-flow direction and the vice versa flow direction is the second air-flow direction; and
wherein, preferably, the aerosol generator provides the small droplets in the inhaling direction; and wherein during inhaling a flow of air flowing around the aerosol generator is provided at a rear side of the aperture surface.

5. Nebulizer head according to claim 4, wherein a portion of the air-flow path between the aerosol generator and the mouth-piece opening is a front air channel (50) and a portion between the aerosol generator and the backside opening is a rear air channel (52); and
wherein the rear air channel and preferably the by-pass sections are equal or larger in clear cross-section than the mouth-piece opening.

6. Nebulizer head according to the preceding claim, wherein the rear air channel is angled upward in the exhaling direction.

7. Nebulizer head according to claim 5 or 6, wherein the mouth-piece is provided as a fixedly mounted mouth-piece.

8. Nebulizer head according to one of the claims 4 to 7, wherein the mouth-piece provides the front air channel having a length (L) between the mouth-piece opening and the aperture member, which length is larger than 5 cm; and
wherein, preferably, the mouth-piece opening is oval in shape.

9. Nebulizer head according to one of the claims 4 to 8, wherein the longitudinal axis of the mouthpiece makes an angle greater than five degrees with respect to an horizontal axis.

10. Nebulizer head according to one of the preceding claims, wherein the aperture member is provided as one of the group of a mesh or membrane with a plurality of holes or apertures, and a plate with a plurality of droplet outlets.

11. A nebulizer system (100), with:
- a nebulizer head (102) according to one of the preceding claims; and
- a base structure (104) comprising a power supply and a control and operating element for operating the aerosol generator;
wherein the nebulizer head is at least temporarily mounted to the base structure.

## Patentansprüche

1. Verneblerkopf (10), bestehend aus:
- eine Gehäusestruktur (12), die einen bidirektionalen Luftströmungsweg (16) zwischen zwei gegenüberliegenden Luftströmungsöffnungen (18a, 18b) bereitstellt, wobei eine dieser Luftströmungsöffnungen als eine Mundstücköffnung und eine andere dieser Luftströmungsöffnungen als eine Rückseitenöffnung vorgesehen ist, und wobei eine Strömungsrichtung von dieser Rückseitenöffnung zu dieser Mundstücköffnung eine erste Luftströmungsrichtung zum Einatmen ist und eine umgekehrte Strömungsrichtung eine zweite Luftströmungsrichtung zum Ausatmen ist;
- einen Flüssigkeitsbehälter (14), der zur Aufnahme einer Flüssigkeit vorgesehen ist, aus der kleine Tröpfchen erzeugt werden sollen, um ein Aerosol zu bilden;
- einen Aerosolgenerator (20) mit einem Öffnungselement (22), das eine Öffnungsfläche (24) mit einer Vielzahl von Öffnungen aufweist, um die kleinen Tröpfchen aus der Flüssigkeit bereitzustellen; wobei das Flüssigkeitsehälter neben dem Aerosolgenerator angeordnet ist, so dass das Öffnungselement mit der Flüssigkeit in Kontakt ist, wobei das Öffnungselement (22) im Wesentlichen vertikal in dem Verneblerkopf (10) in Bezug auf den Luftstromweg (16) montiert ist, der bei normalem Gebrauch des Verneblerkopfes (10) horizontal angeordnet ist; wobei der Aerosolgenerator innerhalb des Luftströmungsweges angeordnet ist; wobei mindestens ein Bypass-Strömungsweg (26) zwischen dem Aerosolgenerator und der Gehäusestruktur vorgesehen ist, wobei der Bypass-Strömungsweg (26) um den Aerosolgenerator (20) herum angeordnet ist, und wobei eine Strömungsrichtung des Bypass-Strömungsweges (26) dieselbe wie die zweite Luftströmungsrichtung ist; wobei die Luft in den Luftströmungsweg eintritt und den Luftströmungsweg über den
Luftstromweg verlässt.
wobei die Öffnungsfläche quer zur ersten und zweiten Luftströmungsrichtung angeordnet ist Strömungsrichtung angeordnet ist; und
wobei der Luftstrom manuell durch das Ein- und Ausatmen des Benutzers aktiviert werden kann und wobei der Luftstrom nicht durch Belüftungsmittel unterstützt wird.

2. Verneblerkopf nach Anspruch 1, **dadurch gekennzeichnet, dass** die Luftströmungsöffnungen jeweils als bidirektionale Lufteinlass- und Luftauslassöffnungen ausgebildet sind.

3. Verneblerkopf nach Anspruch 1 oder 2, wobei der Aerosolgenerator
innerhalb des Luftströmungsweges so angeordnet ist, dass Bypass-Strömungswege um den Aerosolgenerator herum angeordnet sind.

4. Verneblerkopf nach einem der vorhergehenden Ansprüche, wobei eine der Luftströmungsöffnungen als Mundstücköffnung (46) und die andere der Luftströmungsöffnungen als Rückseitenöffnung (48) vorgesehen ist; und eine Strömungsrichtung von der Rückseitenöffnung zur Mundstücköffnung die erste Luftströmungsrichtung und die umgekehrte Strömungsrichtung die zweite Luftströmungsrichtung ist; wobei vorzugsweise der Aerosolgenerator die kleinen Tröpfchen in der Inhalationsrichtung bereitstellt; wobei während des Inhalierens ein Luftstrom, der um den Aerosolgenerator herumströmt, an einer Rückseite der Öffnungsfläche bereitgestellt wird.

5. Verneblerkopf nach Anspruch 4, wobei ein Teil des Luftstromweges zwischen dem Aerosolgenerator und der Mundstücköffnung ein vorderer Luftkanal (50) ist und ein Teil zwischen dem Aerosolgenerator und der rückseitigen Öffnung ein hinterer Luftkanal (52) ist; und wobei der hintere Luftkanal und vorzugsweise die Bypass-Abschnitte den gleichen oder einen größeren lichten Querschnitt als die Mundstücköffnung aufweisen.

6. Verneblerkopf nach dem vorhergehenden Anspruch, wobei der hintere Luftkanal in Ausatmungsrichtung nach oben geneigt ist.

7. Verneblerkopf nach Anspruch 5 oder 6, wobei das Mundstück als fest montiertes Mundstück ausgebildet ist.

8. Verneblerkopf nach einem der Ansprüche 4 bis 7, wobei das Mundstück den vorderen Luftkanal mit einer Länge (L) zwischen der Mundstücköffnung und dem Blendenelement bereitstellt, die größer als 5 cm ist; und wobei die Mundstücköffnung vorzugsweise eine ovale Form aufweist.

9. Verneblerkopf nach einem der Ansprüche 4 bis 8, wobei die Längsachse des Mundstücks einen Winkel von mehr als fünf Grad zu einer horizontalen
Achse bildet.

10. Verneblerkopf nach einem der vorhergehenden Ansprüche, wobei das Öffnungselement als eines aus der Gruppe eines Netzes oder einer Membran mit einer Vielzahl von Löchern oder Öffnungen und einer Platte mit einer Vielzahl von Tröpfchenauslässen vorgesehen ist.

11. Ein Verneblersystem (100), das Folgendes umfasst:
- einen Verneblerkopf (102) nach einem der vorhergehenden Ansprüche; und
- eine Basisstruktur (104) mit einer Stromversorgung und einem Steuer- und Bedienelement zum Betrieb des Aerosolgenerators;
wobei der Verneblerkopf zumindest vorübergehend an der Basisstruktur
befestigt ist.

## Revendications

1. Une tête de nébuliseur (10) comprend :
- une structure de boîtier (12) fournissant un flux d'air bidirectionnel (16) entre deux ouvertures opposées de flux d'air (18a, 18b), où une des ouvertures de flux d'air est fournie comme ouverture de l'embouchure et une autre des ouvertures de flux d'air qui est fourni en tant qu'ouverture arrière, et où la direction du flux de l'ouverture arrière vers l'ouverture de l'embouchure est une première direction du flux d'air pour inhaler et un sens d'écoulement inverse est une seconde direction de flux d'air pour exhaler;
- un réservoir de fluide (14) fourni pour recevoir un liquide à partir duquel des gouttelettes doivent être générées afin de former un aérosol;
- un générateur d'aérosol (20) avec un élément d'ouverture (22) qui comprend une surface d'ouverture (24) avec une pluralité d'ouvertures pour fournir des gouttelettes à partir du liquide;
- où le réservoir de fluide est disposé de manière adjacente au générateur d'aérosol de telle sorte que l'élément d'ouverture est en contact avec le fluide, où l'élément d'ouverture (22) est monté de manière substantielle verticalement dans la tête de nébuliseur (10) par rapport au flux d'air (16) qui dans son utilisation normale de la tête de nébuliseur (10), est disposée de manière horizontale;
où le générateur d'aérosol est disposé à l'intérieur de la voie de l'écoulement d'air;
où au moins un circuit de dérivation (26) est fourni entre le générateur d'aérosol et la structure de boîtier, où le circuit de dérivation (26) est disposé autour du générateur d'aérosol (20), et où une direction d'écoulement du circuit de dérivation (26) est la même que la seconde direction du flux d'air;
où l'air entre dans le flux d'air et quitte le circuit d'écoulement d'air par les ouvertures d'écoulement d'air ;
où la surface d'ouverture est disposée de manière transversale à la première et seconde direction d'écoulement d'air; et
où l'écoulement d'air est activé manuellement par l'utilisateur en inspirant et en expirant respectivement; et où le flux d'air n'est pas soutenu par les moyens de ventilation.

2. La tête de nébuliseur selon la revendication 1, où les ouvertures de flux d'air sont chacune fournies en tant qu'entrée d'air bidirectionnelle et des ouvertures de sorties d'air.

3. La tête de nébuliseur selon la revendication 1 ou 2, où le générateur d'aérosol est disposé à l'intérieur du flux d'air de sorte que les voies d'écoulement de dérivation sont disposées autour du générateur d'aérosol.

4. La tête de nébuliseur selon l'une quelconque des revendications précédentes, où une des ouvertures de flux d'air est fournie en tant qu'ouverture de l'embouchure (46) et l'autre des ouvertures d'écoulement d'air est prévue comme une ouverture arrière (48) ; et une direction d'écoulement allant de l'ouverture arrière à l'ouverture de l'embouchure est la première direction du flux d'air et le sens inverse de l'écoulement est la seconde direction de flux d'air; et où, de préférence, le générateur d'aérosol fournit des gouttelettes dans la direction d'inhalation; et où lors de l'inhalation d'un flux d'air autour du générateur d'aérosol est fournie sur un côté arrière de la surface d'ouverture.

5. La tête de nébuliseur selon la revendication 4, où une partie de la trajectoire du flux d'air entre le générateur d'aérosol et l'ouverture de l'embouchure est un canal d'air frontal (50) et une partie entre le générateur d'aérosol et l'ouverture arrière est un canal d'air arrière (52) ; et
où le canal d'air arrière et de préférence les sections de dérivation sont égaux ou plus grands en section transversale claire que l'ouverture de l'embouchure.

6. La tête de nébuliseur selon la revendication précédente, où le canal d'air arrière est incliné vers le haut dans le sens d'expiration.

7. La tête de nébuliseur selon la revendication 5 ou 6, où l'embouchure est fournie en tant qu'embouchure à montage fixe.

8. La tête de nébuliseur selon l'une quelconque des revendications 4 à 7, où l'embouchure fournit le canal d'air frontal possédant une longueur (L) entre l'ouverture de l'embouchure et l'élément d'ouverture, dont la longueur est supérieure à 5 cm; et où, de préférence, l'ouverture de l'embouchure est ovale.

9. La tête de nébuliseur selon l'une quelconque des revendications 4 à 8, où l'axe longitudinal de l'embouchure forme un angle supérieur à cinq degrés par rapport à l'axe horizontal.

10. La tête de nébuliseur selon l'une quelconque des revendications précédentes, où l'élément d'ouverture est fourni comme l'un des maillages ou une membrane avec une pluralité de trous ou d'ouvertures, et une plaque avec une pluralité des sorties de gouttelettes.

11. Un système de nébuliseur (100), avec :
- une tête de nébuliseur (102) selon l'une quelconque des revendications précédentes; et
- une structure de base (104) comprenant une alimentation électrique et un contrôle et l'élément de commande pour faire fonctionner le générateur d'aérosol;
où la tête de nébuliseur est au moins temporairement fixée à la structure de base.
